# EUROPEAN PATENT SPECIFICATION

(11) **EP 4 483 962 B1**
(45) Date of publication and mention of the grant of the patent: **11.11.2026**
(21) Application number: 24210473.5
(22) Date of filing: 16.12.2020
(51) Int. Cl.: A61L 15/46, A61K 38/10, C07K 7/08, A61P 31/02, A61P 31/04

(54) **IMPROVED WOUND CARE COMPOSITION AND METHODS**
VERBESSERTE WUNDPFLEGEZUSAMMENSETZUNG UND VERFAHREN
COMPOSITION ET PROCÉDÉS DE SOIN DES PLAIES AMÉLIORÉS

(30) Priority: 18.12.2019 SE 1951495
(43) Date of publication of application: 01.01.2025
(62) Document number(s) of the earlier application(s) in accordance with Art. 76 EPC: 20842349.1 / 4 076 549
(73) Proprietor: Amferia AB, 431 83 Mölndal (SE)
(72) Inventor: KUMAR RAJESKHARAN, Anand, 412 60 Göteborg (SE); ATEFYKTA, Saba, 412 59 Göteborg (SE); ANDERSSON, Martin, 431 38 Mölndal (SE); BLOMSTRAND, Edvin, 438 95 Hällingsjö (SE)
(74) Representative: Henricson Briggs, David James

(56) References cited:
- WO-A1-2019/074422
- AGNIESZKA K MUSZANSKA ET AL: "Pluroniclysozyme conjugates as anti-adhesive and antibacterial bifunctional polymers for surface coating", BIOMATERIALS, ELSEVIER, AMSTERDAM, NL, vol. 32, no. 26, 5 May 2011 (2011-05-05), pages 6333 - 6341, XP028097289, ISSN: 0142-9612, [retrieved on 20110511], DOI: 10.1016/J.BIOMATERIALS.2011.05.016

## Description

### Field of the Invention

The present disclosure relates to antimicrobial hydrogels. Specifically, it relates to an antimicrobial amphiphilic hydrogel composition for use in haemostasis of a wound. The antimicrobial hydrogel may be provided as a dispersion.

### Background of the invention

Wound infection involving the skin or tissues in the vicinity of a wound interferes with the healing process and may cause systemic illnesses. Today, antibiotic therapy is the most common treatment for treating wound infection. Over many years, such antibiotic therapy routines have not only been shown to cause systemic side effects to the patients but has also resulted in a rapid increase in severe infections caused by antibiotic resistant bacteria.

Haemostasis or hemostasis is the body's process to prevent and stop bleeding. Haemostasis involves blood coagulation and the formulation of blood clots to stop bleeding. It is known in wound-care to use Microfibrillar collagen haemostats to accelerate the formation of blood clots. Microfibrillar collagen is available as a sheet, a powder and as a sponge.

The commercially available wound dressings such as Mepilex^{®} or Mepilex-Ag^{®} (marketed by Mölnlycke Health Care) incorporate soft superabsorbent dressing layers comprising silver as the antimicrobial agent. The silver is released into the wound and kills the microbes by damaging the cell wall or inhibiting the microbe from reproduction. Numerous other wound dressings incorporate antimicrobial molecules such as chlorhexidine or conventional antibiotic drugs such as penicillin are used to prevent bacterial adhesion or infection at the wound site. However, the above compounds are limited in use due to their limited spectrum of activity, cytotoxicity to human cells and the possibility of development of antimicrobial resistance after only a short period. Furthermore, the release of silver into the water system has detrimental environmental effects. According to Alternative drinking-water disinfectants: bromine, iodine and silver. World Health Organization; 2018 "Based on the lowest median L(E)C50 value of the key environmental organisms both silver salt and silver nanoparticles would be classified as "very toxic to aquatic organisms" under EU Directive 93/67/EEC (CEC, 1996)"

Generally wound care devices such as those named above, which may have antibacterial characteristics, simply collect, or soak up blood.

WO 2019/074422 A1 (AMFERIA AB) 18 April 2019 describes an antimicrobial amphiphilic hydrogel. The hydrogel of WO 2019/074422 A1 is disclosed as a solid singular material suitable for use as an antimicrobial wound-care device, especially in antibiotic resistant infections. This document does not relate to haemostasis or bleeding wounds. Improved compositions and new uses of the material may improve wound-care alternatives for medical personal and patients.

Muszanska, K A et al. Pluronic-lysozyme conjugates as anti-adhesive and antibacterial bifunctional polymers for surface coating. 2011, Vol. 32, Issue 26 describes antibacterial polymer brushes formed by non-crosslinked PEO-PPO-PEO triblock copolymer micelles.

Devices which have a combined antimicrobial and haemostatic effect would be advantageous.

### Summary of the invention

Accordingly, the present invention preferably seeks to mitigate, alleviate or eliminate one or more of the above-identified deficiencies in the art singly or in any combination and solves at least the above-mentioned problems by providing an antimicrobial amphiphilic hydrogel composition comprising: a first cross-linkable amphiphilic component, the first amphiphilic component, in its chemically cross-linked state, being a lyotropic liquid crystal and having an ordered nanostructure of hydrophobic and hydrophilic domains, an antimicrobial agent covalently attached to the hydrophilic and/or hydrophobic domains, wherein the cross-linkable amphiphilic component is a cross-linked lyotropic liquid crystal, and wherein the composition is a suspension of cross-linked hydrogel particles in a solution.

As opposed to other antimicrobial haemostatic devices the amphiphilic hydrogel is non-biodegradable and the antimicrobial agents are covalently attached to the hydrogel which means that it will not leach hydrogel and/or antimicrobial agents in to the wound and is long-term stable. Surprisingly rather than modifying a Microfibrillar collagen device the present inventors have used a new material which appears to have a combined antimicrobial and haemostatic effect. Furthermore, the provision of an antimicrobial amphiphilic material allows the material to absorb both hydrophobic and hydrophilic substances such as bacterial toxins released from dying bacteria. The small and repeating domains furthermore enables a higher density of antimicrobial agents to be present on the surface of the hydrogel. Compared to previous antimicrobial haemostatic devices the present amphiphilic hydrogel is capable of significant swelling from a wet state and therefore blood exudate and blood can absorb into the hydrogel.

Further, an antimicrobial hydrogel composition for use in haemostasis of a wound, wherein the antimicrobial agent is a proline arginine-rich end leucine-rich repeat protein, PRELP, derived antimicrobial peptide, such as an antimicrobial peptide having a 90-95% identity to RRPRPRPRP is provided. Such proteins have been shown to be similar to human cathelicidin derived LL-37 with respect to several aspects, such as net charge and antimicrobial efficacy (Malmsten, M et al., Highly Selective End-Tagged Antimicrobial Peptides Derived from PRELP, PLoS ONE, 2011, 6(1): e16400. doi:10.1371/journal.pone.0016400). However, LL-37 has previously been shown not to affect plasmatic coagulation (Harm, S et al., Blood Compatibility - An Important but Often Forgotten Aspect of the Characterization of Antimicrobial Peptides for Clinical Application, Int. J. Mol. Sci. 2019, 20, 5426; doi:10.3390/ijms20215426).

The dispersion of particles has advantages such as improved wound coverage of irregular shaped wounds, a greater surface area available for the attachment of antimicrobial agents, easier and quicker application compared to previous solid hydrogels. The particles have been furthermore shown to maintain their antimicrobial function in particle form. The particles absorb both aqueous and non-aqueous solutions.

A method of preparing a dispersion comprising antimicrobial amphiphilic hydrogel particles is provided.

Devices having a coating of the dispersion of antimicrobial amphiphilic hydrogel particles are also disclosed.

Due to the sprayable nature of the particle dispersion coating devices for use with human bodies or bodily fluids is simplified.

Uses of the dispersion are also disclosed.

Furthermore, the composition for use in a method of treating a wound is provided.

A proline arginine-rich end leucine-rich repeat protein, PRELP, derived antimicrobial peptide, such as an antimicrobial peptide having a 90-95% identity to RRPRPRPRP, such as RRP9W4 for haemostasis of a wound is provided.

Further advantageous embodiments are disclosed in the appended and dependent patent claims.

### Brief description of the drawings

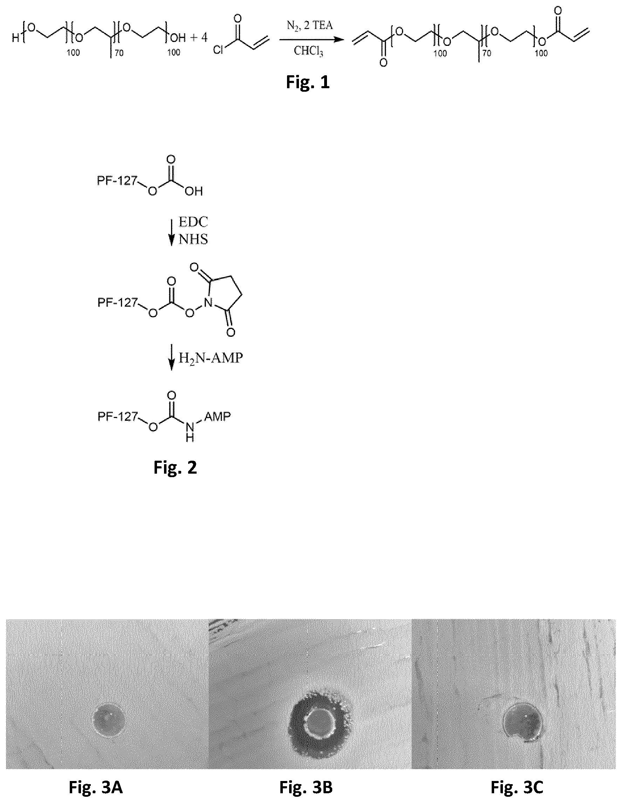

These and other aspects, features and advantages of which the invention is capable will be apparent and elucidated from the following description of embodiments of the present invention, reference being made to the accompanying drawings, in which
Fig. 1 is a synthesis scheme of diacrylate modified Pluronic^{®} triblock copolymer where X and Y refer to the number of PEO and PPO groups.
Fig. 2 shows the reaction scheme of the covalent attachment of antimicrobial peptides to diacrylate modified Pluronic triblock copolymer F-127 by EDC/NHS activation.
Fig. 3 shows a zone inhibition test of the antimicrobial hydrogel vs control samples. Fig 3a shows a negative control amphiphilic hydrogel with no AMPs; Fig 3b shows an amphiphilic hydrogel with only physically absorbed AMPs; Fig 3c shows amphiphilic antimicrobial hydrogel according to an aspect where AMPs are covalently attached to the amphiphilic hydrogel. The zone of inhibition is the darker region and can be seen extending beyond the area of the hydrogel (the central circular element), whereas in Fig 3C the zone of inhibition is directly below the hydrogel, indicating that no AMP has leached from the hydrogel.

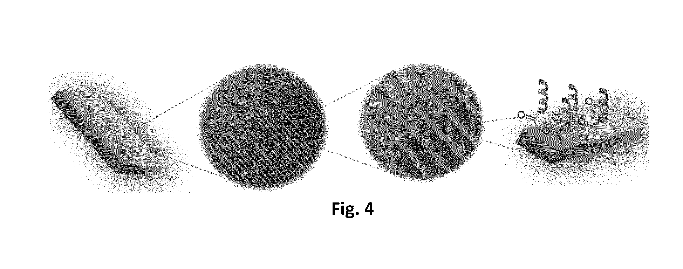

Fig. 4 shows a schematic of covalently bonded AMPs to a chemically cross-linked amphiphilic hydrogel with a repeating, 3D printed and aligned, normal hexagonal ordered nanostructure.

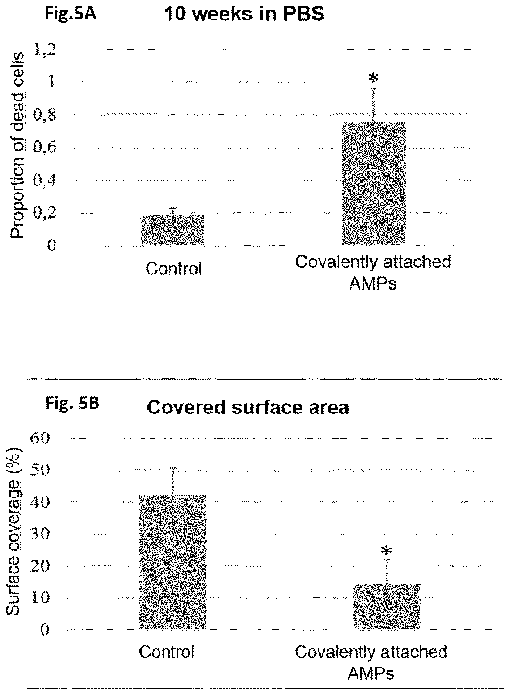

Fig. 5 shows the results from a storage stability test in phosphate buffered saline (PBS). In Fig. 5a the proportion of dead cells (*S. aureus*) can be viewed for a control amphiphilic hydrogel and an antimicrobial amphiphilic hydrogel. Fig. 5b shows the total surface coverage found on the hydrogels. The asterisk (*) indicates a significant difference compared to the control sample at a 95% confidence level.

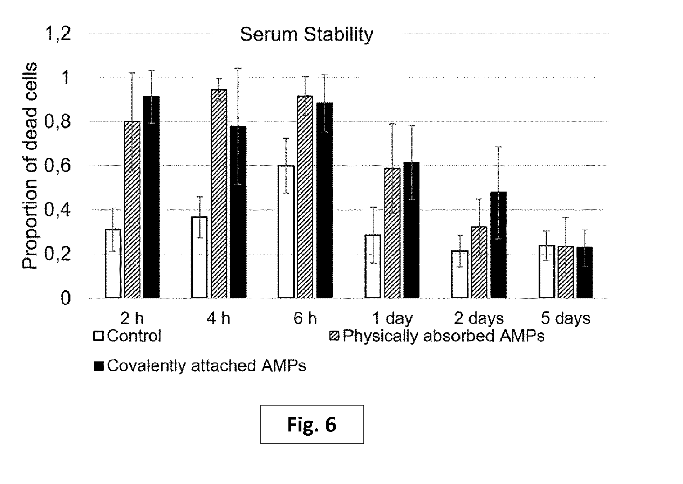

Fig. 6 shows the results from a serum stability test where the hydrogels were exposed to 20% human serum. The hydrogels were taken out from the serum at the times indicated on the x-axis. The proportion of dead cells (*S. aureus*) on the surface of the hydrogels were determined by live/dead staining and is found on the y-axis. At each time point, except at 5 days, there was a significant difference at a 95% confidence level between the activated surfaces compared to the control. Each bar is compiled of images taken of four samples.

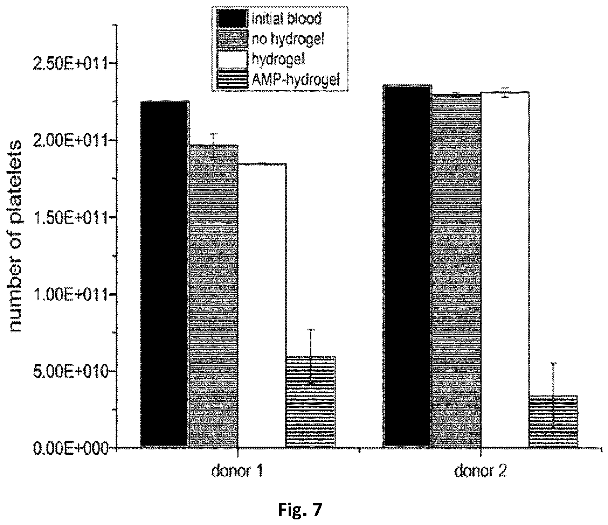

Fig. 7 shows the result of the blood coagulation experiment (Experiment 1). The figure shows, for two donors, the number of platelets after whole blood incubation at 37° for 60 min for; initial blood, blood incubated without contact to the hydrogel, contact with the amphiphilic hydrogel, blood incubated with the antimicrobial amphiphilic hydrogel. Data represent mean ± standard error of the mean for n= 4.

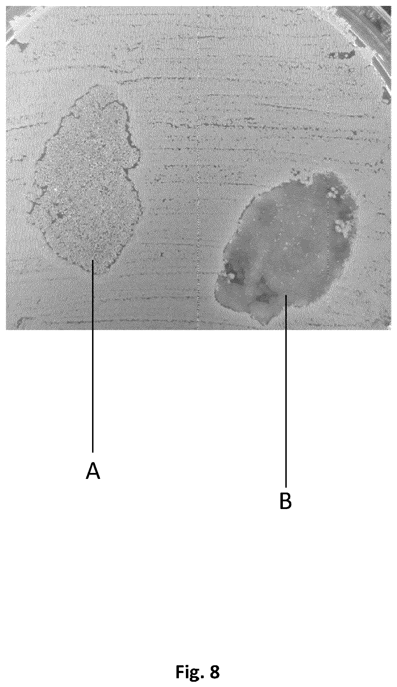

Fig. 8 shows control and AMP-activated particles placed upon agar plates streaked with *S. aureus* and cultivated overnight (approximately 15 hours). Region A is the control region (hydrogel with no AMP) and region B is the AMP-activated hydrogel particles.

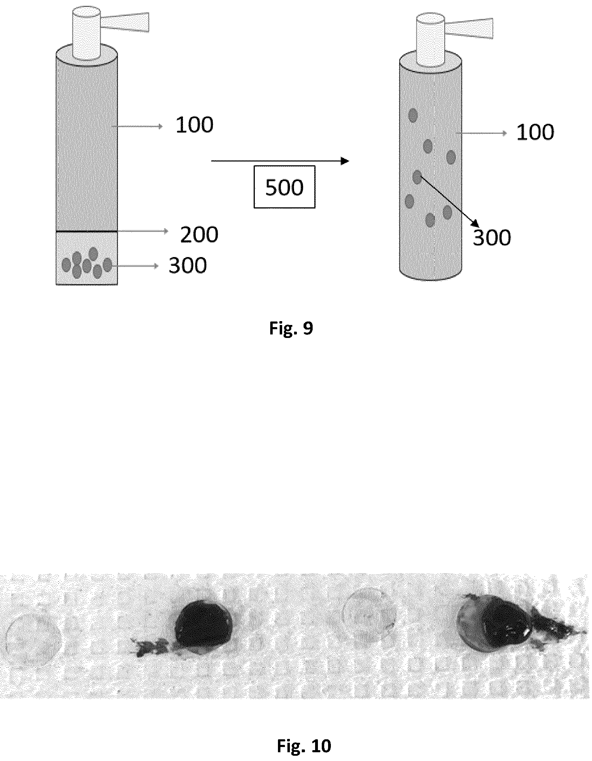

Fig. 9 shows a schematic diagram of a spray device according to an aspect.
Fig. 10 shows an image of blood coagulation tests (Experiment 1) with human blood on amphiphilic hydrogels with and without AMPs. From left to right: donor 1 blood on hydrogel with no AMP, donor 1 blood on hydrogel with AMP, donor 2 blood on hydrogel with no AMP, donor 2 blood on hydrogel with AMP. The coagulated blood can clearly be seen in the two hydrogels with AMP samples.

### Detailed description

The following description of the present invention describes antimicrobial amphiphilic hydrogels, uses of antimicrobial amphiphilic hydrogels in wound-care, and compositions comprising such antimicrobial amphiphilic hydrogels. The antimicrobial hydrogel comprises a first cross-linkable amphiphilic component. In its cross-linked state, the amphiphilic component results in a hydrogel comprising an ordered structure of hydrophilic and hydrophobic domains. The antimicrobial hydrogel further comprises an antimicrobial agent covalently bonded to the repeating hydrophilic and/or hydrophobic domains of the cross-linked hydrogel. Details regarding methods of manufacture of the amphiphilic hydrogel and its benefits are detailed in WO 2019/074422 A1.

The repeating ordered nanostructure of the amphiphilic hydrogel comprises repeating and alternating hydrophobic-hydrophilic domains. The morphology and specific structure of the hydrophobic-hydrophilic domains is discussed below. The hydrogel comprises an ordered and repeating nanostructure throughout the hydrogel, that is, not only on the surface of the hydrogel. The cross-linked hydrogel is solid. Intermolecular cross-linking irreversibly locks the ordered structure and results in a hydrogel which has a high integrity and is mechanically resilient.

The antimicrobial hydrogel is especially suitable for wound care applications due to the ordered and repeating nanostructure leading to an ordered and repeating provision of antimicrobial agents on the skin or wound surface. Furthermore, antimicrobial agents are better immobilized leading to better long-term performance.

The hydrogel may be considered to form a substrate on to which antimicrobial agents can be immobilized. The hydrogel is, in its cross-linked state, self-supporting and three-dimensional. Generally, the hydrogel is substantially non-degrading in physiological conditions. That is, the hydrogel is non-biodegradable, it is substantially not degraded by chemical or enzymatic conditions found in the relevant *in vitro* and *in vivo* conditions. For example, the hydrogel is not degraded in the presence of blood, sweat, urine or other biological fluids. Furthermore, the hydrogel is substantially non-degrading, that is, it is stable and remains a solid, in relatively low or high pH environments.

The hydrogel may form a first substantially uniform layer to which antimicrobial agents are covalently attached. Antimicrobial agents may be covalently attached to at least the repeating hydrophilic domains of the hydrogel throughout the layer. They may be provided within the layer, and not only on the surface of the hydrogel. This is a significant improvement compared to techniques of surface modification of a hydrogel or other substrate which lead to only surface immobilization of antimicrobial agents. The small and repeating domains furthermore enable a higher density of antimicrobial agents to be present on the surface of the hydrogel

The repeating and ordered structure of hydrophilic and hydrophobic domains is ordered on at least the nanoscale, and as will be discussed further below, may be ordered on a larger, micro or macro scale depending on production techniques. The terms ordered and repeating relate to the hydrogel having a defined periodicity. As opposed to hydrogels based on carbohydrates, polysaccharides or other non-amphiphilic molecules the nanostructure of the hydrogel as described herein has an ordered and repeating nanostructure and is not randomly cross-linked. The hydrogel is amphiphilic. After cross linking, the amphiphilic hydrogel is a chemically cross-linked amphiphilic hydrogel.

The ordered and repeating nanostructure results in antimicrobial agents covalently bonded to the hydrogel having a defined orientation. If the antimicrobial agents themselves are amphiphilic then the antimicrobial agent is furthermore more effectively immobilized on the surface of, and within, the hydrogel. The amphiphilicity of the hydrogel also allows the absorption of both aqueous and non-aqueous solutions.

As stated above, as opposed to a hydrogel having a surface treatment to define a surface chemistry, the hydrogel of the present disclosure has a repeating and ordered nanostructure both on the surface and within the bulk of the hydrogel. This leads to improved immobilization of antimicrobial agents both on the surface and within the hydrogel.

The antimicrobial hydrogel may be formed by the chemical cross-linking of organic amphiphilic materials such as cross-linkable copolymers, cross-linkable surfactants, cross-linkable proteins, cross-linkable peptides and cross-linkable lipids. Cross-linkable as used herein refers to the covalent linkage of molecules to each other using reactive chemical groups present on the molecules. The chemical cross-linking process can be catalysed using light such as ultraviolet light, heat, or other chemical catalysts such as enzymes. The covalent cross-linking of the hydrogel is non-reversible. The covalent cross-linking will not degrade or disintegrate at increased temperatures. The covalent cross-linking is also stable towards pH variations.

The first amphiphilic component of the hydrogel may be a cross-linkable amphiphilic polymer. A typical and suitable amphiphilic material is a diacrylate modified poloxamer, such as, polyethylene oxide-polypropylene oxide-polyethylene oxide (DA-PEOₓ-PPO_{y}-PEOₓ-DA, where x and y refer to the number of PEO and PPO groups present respectively) as described in the experimental section below. Specifically, the amphiphilic material may be the amphiphilic tri-block co-polymers, polyethylene oxide(100)-polypropylene oxide(70)-polyethylene oxide(100) (Pluronic^{®} F127 - BASF Corporation), polyethylene oxide(30)-polypropylene oxide(70)-polyethylene oxide(30) (Pluronic^{®} P123 - BASF Corporation).

As stated above, the amphiphilic component may be a diacrylate derivative of a tri-block copolymer thus enabling the copolymer to be chemically cross-linked. A process for diacrylate modification is provided in the experimental section below. The modification may be performed via the reaction of a triblock amphiphilic copolymer with acryloyl chloride to form a diacrylate derivative. Other methods of forming cross-linkable amphiphilic polymers may be possible such as forming methacrylate derivatives or via carboxylic-amine bridges.

The cross-linkable amphiphilic polymer may, in the presence of water, self-assemble to form ordered nanostructures called lyotropic liquid crystals (LLC). In its cross-linked form, that is, after cross-linking, the hydrogel may be considered a chemically cross-linked lyotropic liquid crystal (LLC). The cross-linking of the amphiphilic polymer may be considered to form a polymerized lyotropic liquid crystal (PLLC) having a well-defined structure.

A non-solid cross-linked hydrogel may have a structure of spherical micellar aggregates in size range of 2-100 nm arranged randomly throughout the hydrogel called as a normal micellar system, denoted in shorthand as L₁. Such a normal micellar hydrogel may comprise from about 1% to about 19% (%wt) amphiphilic polymer, and from about 99% to about 81% (%wt) water. Generally, this system does not form a cross-linked solid gel, however in certain cases such as between the range of 15-19% (%wt) amphiphilic polymer concentration, the system can exist as a cross-linked solid with very soft and pliable mechanical features.

The hydrogel may have a structure of spherical micellar aggregates in the size range 2-100 nm arranged in a lyotropic liquid crystal, cubic shaped, ordered arrangement known as a normal micellar cubic system, denoted in shorthand as I₁, with a primitive arrangement (P...) or body centred (B...) arrangement or a face centred (F...) arrangement of micellar structures in a cubic lattice. An example of a normal micellar cubic structure with an Im3m crystal symmetry may comprise from about 20% to about 65% (%wt) amphiphilic polymer and from about 80% to about 35% (%wt) water. Another example composition to obtain a normal micellar cubic system with primitive arrangement of a micellar structures in a cubic lattice is 65% (%wt) water, 10% (%wt) butanol and 25% (%wt) amphiphilic polymer.

The hydrogel may have a structure of spherical micellar aggregates in the size range 2-100 nm arranged in a lyotropic liquid crystal, bicontinuous cubic shaped, ordered arrangement known as a micellar cubic system with Pn3m crystal structure. Such a bicontinous micellar cubic system with Pn3m crystal structure may comprise from about 25% to about 65% (%wt) amphiphilic polymer and from about 75% to about 35% (%wt) water. Another example composition to obtain such a LLC structure is 33-38% (%wt) water and the rest composed of an amphiphilic species or amphiphilic polymer.

The hydrogel may have a structure of spherical micellar aggregates in the size range 2-100 nm arranged in a lyotropic liquid crystal, bicontinuous cubic shaped, ordered arrangement known as a micellar cubic system with Ia3d crystal structure. An example composition to obtain such a LLC structure is 13-32% (%wt) water and the rest composed of the amphiphilic species or amphiphilic polymer.

The hydrogel may have a structure of cylindrical micellar aggregates with diameter of cylinders in the size range of 2-100 nm arranged in an ordered lyotropic liquid crystal, hexagonal geometry called as a normal hexagonal system In such a normal hexagonal system, the amphiphilic polymer may be present in from about 30% to about 80% (%wt) and water may be present in from about 60% to about 20% (%wt), with or without minor amounts of organic solvents. Such a normal micellar hexagonal system may comprise from about 35% to about 40% (%wt) amphiphilic polymer, about 50% (%wt) water, and from about 10% to about 15% (%wt) organic solvent.

The antimicrobial hydrogel may also have a chemically cross-linked, ordered nanostructure of the following structures with a neutral geometry and zero curvature; sheet-like micellar aggregates with distance between adjacent sheets is in the range of 2-100 nm, arranged as lyotropic liquid crystal, lamellar geometry called as a lamellar system. Such a lamellar system might comprise anywhere between 20-80% (%wt) amphiphilic molecule, 15-60% (%wt) aqueous solution and 0-25% (%wt) organic solvents such as butanol. An example composition of to obtain a lamellar LLC is 20% amphiphilic polymer, 55% (%wt) water and 25% (%wt) organic solvent such as butanol.

Micellar and lyotropic liquid crystal nanostructures of the antimicrobial hydrogel may comprise aqueous liquids such as water as the continuous domain and hydrophobic parts confined within the micellar aggregates. The micellar and lyotropic liquid crystal nanostructure may comprise an aqueous liquid such as water confined within the micellar aggregates and a hydrophobic continuous domain. Aqueous liquids include, but are not limited to, water, salt solutions, blood, sweat and other possible biological fluids. In its fully wet, also known as swollen state, the antimicrobial hydrogel can absorb up to 3 to 4 times its own weight of aqueous liquids. A fully wet/swollen state refers to the original concentration (by weight) of the hydrogel of 20-90% aqueous solution and 10-80 % amphiphilic organic molecules, depending on the type of cross-linked LLC structure the hydrogels possesses. In its fully dry state the hydrogel uniformly contains less than 10 % aqueous solution by weight, and more usually less than 5 % aqueous solution by weight, in which case it may absorb up to 8 to 10 times aqueous solution of its own weight. Following liquid absorption, the antimicrobial hydrogel swells and changes in size. However, the shape and geometry of the hydrogels is substantially retained.

Due to the amphiphilicity of the antimicrobial hydrogel it may also absorb hydrophobic liquids. In the presence of the hydrophobic solvent chloroform, the fully-dry hydrogel can absorb hydrophobic liquid, such as chloroform, up to 20 to 30 times its own weight. As above, a fully dry state refers to the concentration of the hydrogel of less than 5 % aqueous solution and greater than 95 % amphiphilic organic molecules by weight. The fully dry state does not refer to a freeze-dried polymer network, which cannot be considered a hydrogel.

The liquid absorption properties of the hydrogels can be tailored to absorb more or less water or hydrophobic liquids. This can be achieved by using amphiphilic molecules of different ratios of chain lengths in hydrophilic to hydrophobic groups to form the hydrogel. For example, amphiphilic block copolymer DA-PEOₓ-PPO_{y}-PEOₓ-DA, where x and y refer to the number of PEO and PPO, groups can possess more or less PEO or PPO groups. Higher amounts of PEO groups than PPO groups may result in a hydrogel with high water absorption capacity, up to 3 to 8 times its own initial weight. Conversely, a hydrogel with more PPO groups than PEO groups absorbs less water approximately, 0.5-1.5 times its initial weight.

The antimicrobial agent is covalently bonded to the repeating hydrophilic and/or hydrophobic domains. In the antimicrobial hydrogel, there is a plurality of antimicrobial molecules each of which is covalently bonded to at least a portion of the repeating and periodic hydrophilic and/or the hydrophobic domains.

Greater than 10%, such as greater than 50%, or greater than 90% of the antimicrobial agent present in the hydrogel may be covalently attached to the hydrogel. This results in greater stability and reduced leaching of antimicrobial agent from the hydrogel.

The antimicrobial agent may be an amphiphilic antimicrobial agent. That is, the antimicrobial molecule may have a hydrophilic region and a hydrophobic region. The antimicrobial agent may be selected such that it ruptures the bacterial cell wall via electrostatic forces. The antimicrobial agent may be an antimicrobial polymeric molecule such as polymeric biocides or an antimicrobial peptide (AMP). AMPs generally disrupt or inhibit microbial growth and proliferation by damaging the microbes' cell membranes. AMPs are generally amphiphilic. AMPs are generally short chain peptides, i.e. consisting of 1-50 amino acids, and molecular weights between 1-50 kDa. AMPs can be linear chained AMPs, branched AMPs and/or cyclic AMPs. They generally possess a net positive charge and possess both hydrophilic and hydrophobic regions. It is known that the positively charged, amphiphilic structure of AMPs enables the peptide to penetrate the negatively charged bacterial membrane. The compromised cell wall leads to cell death. The amphiphilic nature of AMPs in combination with the ordered and repeating nanostructure of the hydrogel leads to orientation and higher immobilization of AMPs. That is, the AMPs do not separate or release from the underlying hydrogel. This results in the antimicrobial hydrogel being a non-leachable substrate for the antimicrobial agents. An AMP may be covalently attached to both a hydrophilic domain and a hydrophobic domain of the amphiphilic hydrogel. An AMP may be covalently attached to adjacent hydrophilic and hydrophobic domains. The N-terminus of an AMP may be covalently attached to the hydrophobic domains of the hydrogel. The C-terminus of an AMP may be covalently attached to the hydrophilic domains of the hydrogel.

An AMP may be both covalently attached to the amphiphilic hydrogel and physically absorbed in to the hydrogel. As is shown by the right-most image in Fig. 3B of WO 2019/074422 A1 even after 3 weeks of washing in 50% ethanol the amphiphilic hydrogel does not release all of the physically absorbed fluorescent tagged AMP. This is due to the amphiphilicity of the hydrogel and the interaction of the AMP with the hydrophilic and hydrophobic domains of the hydrogel. This results in increased antimicrobial performance and long-term stability during use.

The antimicrobial agent may be silver (Ag). For example, the antimicrobial agent may be a silver nanoparticle immobilized within or on the repeating and ordered hydrophilic and/or hydrophobic domains of the hydrogel. It is known that, silver has the disadvantage of increased toxicity to mammalian cells and has detrimental environmental effects if it is, for example, released into the water system, however, it is also generally a lower cost antimicrobial agent in comparison to an AMP.

It would not be apparent to the skilled person that carboxyl groups would be present in the amphiphilic hydrogel. Therefore, there is no reason as such to attempt to covalently attach an AMP to the amphiphilic hydrogel, without additional modifications to the hydrogel.

The immobilization is generally achieved via the covalent bonds between carboxyl groups on the hydrophilic domains of the hydrogel. In the case of the antimicrobial agent being an antimicrobial peptide, strong amide bonds are formed between the AMP and the repeating hydrophilic domains of the hydrogel. The AMP may be covalently bonded to the hydrogel via 1-Ethyl-3-(3-dimethylaminopropyl) carbodiimid (EDC)-N-hydroxysuccinimide (NHS) activation of the carboxyl groups present on the hydrophilic domains of the hydrogel. The reaction scheme for such covalent attachment of AMPs via EDC/NHS activation can be seen in Fig 2. AMPs or other antimicrobial agents may furthermore be physically absorbed in to the hydrogel, however, in such a case there is no covalent bonding of the antimicrobial agent to the hydrophilic or hydrophobic regions of the hydrogel. Non-covalently bonded antimicrobial agents are, however, prone to relatively faster degradation and leaching/release from the hydrogel. In the zone inhibition test shown in Fig. 3 it can be seen that physically absorbed AMPs tend to leach/release from the hydrogel, whereas covalently bonded or attached AMPs do not leach.

The AMP, or other amphiphilic antimicrobial agent, has a hydrophobic region which interacts with the ordered and repeating hydrophobic regions of the hydrogel. This leads to improved orientation and improved immobilization of the antimicrobial agent. Stability and resistance to degradation of the AMPs is thus increased, while decreasing or eliminating the release of AMPs in to the surrounding environment, due to the hydrogel having ordered and repeating hydrophilic and hydrophobic domains. Such an architecture is contended to improve the stability and activity of AMPs.

As shown in the experimentation section below, the antimicrobial hydrogel is capable of killing up to 99.99% of bacteria. Without being bound by theory, a further benefit is that the hydrophilic domains of the antimicrobial hydrogel are capable of attracting the negatively charged bacteria and therein effectively killing them. In wound care applications, this may also lead to the removal of dead and/or attached bacteria via removal of a wound dressing comprising the antimicrobial hydrogel. The experimental results for gram-positive and gram-negative bacteria suggest that the antimicrobial hydrogel is also capable of killing drug resistant strains of bacteria such as MRSA and multi-drug resistant (MDR) *E-coli.*

As shown in the experimental section, the antimicrobial peptide may be one or more of the following; RRPRPRPRPWWWW-NH2 (RRP9W4N, Red Glead Discovery AB, Lund, Sweden), RRPRPRPRP-NH2 (RRP9N, Red Glead Discovery AB, Lund, Sweden), RRPRPRPWWWWRP-NH2 (RRP7W4RPN, Red Glead Discovery AB, Lund, Sweden), RRPRPWWRPWWRP-NH2 (RRP5W2RPW2RPN, Red Glead Discovery AB, Lund, Sweden). Sequences for RRP9W4N, RRP9N, are provided in WO 2012/033450 A1. Sequences for RRP7W4RPN and RRP5W2RPW2RPN are provided in WO 2019/074422 A1. The antimicrobial peptide may be an antimicrobial peptide comprising less than 20 amino acids which comprises an amino acid sequence having at least 90%, such as 95%, identity to the amino acid sequence RRPRPRPRP (sequence provided in WO 2012/033450 A1), and optionally, a stretch of at least three consecutive tryptophan or phenylalaline residues appended to either the C- or N-terminus, or therebetween. The antimicrobial peptide may comprise an N-terminal amidation. The antimicrobial peptide may be an antimicrobial peptide comprising a stretch of at least one, such as at least three, hydrophobic amino acids, such as phenylalanine, or tryptophan residues, forming a hydrophobic region. The hydrophobic region allows for interaction with the hydrophobic regions of the hydrogel. However, other antimicrobial peptides may be suitable for use as the antimicrobial agent.

The antimicrobial agent may be a synthetically derived AMP as those in the preceding paragraph or biologically derived. Biologically derived AMPs can be derived from a kininogen proteins, proline and arginine rich end leucine rich repeat protein (PRELP), growth factor proteins, coagulation system proteins, complement factor C3a, von Willebrand factor, vitronectin, superoxide dismutase, prion proteins, protein C inhibitor, fibronectin, laminin, chemokines, and histidine rich glycoprotein. Some examples of biologically derived AMPs are human cathelicidin derived LL-37 peptide and Omiganan pentahydrochloride. All these peptides can be potentially incorporated in the hydrogel as covalently attached or physically absorbed. Alone or together with other peptides. The antimicrobial agent may be an LL-37 derivative having less than 37 amino acids, that is, a length of less than the wild-type LL-37. The antimicrobial agent is preferably a proline arginine-rich end leucine-rich repeat protein (PRELP) derived peptide. Preferably the antimicrobial agent is comparable to LL-37 in charge and antimicrobial efficacy. As described in the experimental section the antimicrobial agent is preferably RRP9W4 which is a PRELP derived peptide comparable to LL-37 with respect to antimicrobial efficacy and net charge.

The antimicrobial agent may be attached to the hydrogel via a variety of processes. As is shown in the experimental section the antimicrobial agent may be attached via immersion of the hydrogel in a solution comprising the antimicrobial agent. An antimicrobial agent may be applied substantially to the surface of the hydrogel via a surface application process as opposed to immersion. A solution comprising the antimicrobial agent may be dropped on to the surface of the hydrogel. A solution comprising the antimicrobial agent may be sprayed on to the hydrogel. As is shown in the experimental section the amount of antimicrobial agent required for antimicrobial activation of the surface is generally reduced significantly via dropping and spraying compared to immersion. This is because the bulk of the hydrogel is not activated with an antimicrobial agent.

In addition to the antimicrobial agent the hydrogel may comprise at least one therapeutic agent. Due to the ordered and repeating hydrophilic and hydrophobic domains of the hydrogel the therapeutic agent may be hydrophobic, hydrophilic, or amphiphilic, polar or non-polar. The antimicrobial hydrogel can host hydrophobic therapeutic agent(s) in the hydrophobic domain while the hydrophilic domains will host hydrophilic therapeutic agent(s). A therapeutic agent can be, but not limited to, drug molecules or small biomolecules such as peptides or proteins with anti-inflammatory, antibiotic or anticancer properties. This property of selective release of therapeutic agents from the antimicrobial hydrogels can, in addition to its antimicrobial properties, be used in medical devices such as for wound care and wound healing or other antimicrobial/drug release applications. At least one therapeutic agent may be covalently attached or physically absorbed to the hydrophobic and/or hydrophilic domains of the antimicrobial hydrogel. A plurality of therapeutic agents may be provided to the hydrogel. In such cases a first therapeutic agent may be covalently attached to the hydrogel and a second, third, etc. therapeutic agent may be physically absorbed. As opposed to the antimicrobial agent, the at least one therapeutic agent need not be immobilized on or within the hydrogel but may be free to substantially leach from the surface.

The antimicrobial hydrogel does not adhere or stick to biological surfaces like skin or a wound bed. This leads to improved performance in a variety of applications. A wound care article such as a wound dressing must be soft and be able to absorb the excess wound exudate in order to contain infection and keep the wound environment from harbouring microbes. The antimicrobial hydrogel can be used as a wound dressing to absorb uncontrollable exudates released from a compromised skin. Wound exudates can contain pus, blood, water, and sweat. Due to the high and versatile absorption properties of the antimicrobial hydrogel, in combination with the antimicrobial properties it is especially suitable as a wound care article. The antimicrobial hydrogel absorbs substantially the same amount of water compared to a hydrogel, comprising amphiphilic component, but without an antimicrobial agent covalently bonded thereto. The antimicrobial hydrogel therefore has sufficient wound exudate absorbing performance even when comprising an antimicrobial agent. The amphiphilicity of the antimicrobial hydrogel furthermore allows the material to absorb both hydrophobic and hydrophilic substances such as bacterial toxins released from dying bacteria.

The antimicrobial amphiphilic hydrogel is prepared as a dispersion of particles in a continuous medium, wherein the particles are present as a suspension of particles in a solution. The solid cross-linked amphiphilic hydrogel is processed via grinding or the like to obtain a collection of amphiphilic hydrogel particles. The particles may have a diameter of from about 0.01 mm to about 0.5 mm. As the particles may be formed via, for example, a grinding process, the particles need not be regular spherical particles but may be irregular. Particles being smaller than the range identified above may also be obtainable via other production methods. For example, particles in the size range of 10 nm to 1 µm in diameter may be possible to obtain via a dispersing method such as via sonication.

An alternative but not claimed process for obtaining the particles is to first disperse the non-crosslinked polymer in solution prior to cross-linking. For example, the non-crosslinked amphiphilic polymer may be provided to a solution e.g., water forming an LLC. The LLC hydrogel formed in the water may then be rapidly dispersed in solution via, e.g., blade mixing and subsequently UV cross-linked. In summary, particles may range in size from 10 nm to 0.5 mm depending on the process used for forming the particles.

The particles may be present in dry form or may be swollen with for example, an aqueous solution. The particles of amphiphilic hydrogel may be thereafter functionalised with an antimicrobial agent such as an AMP. As described above, the antimicrobial agent may be covalently attached to at least the hydrophilic regions of the hydrogel. The antimicrobial agent may be an amphiphilic antimicrobial agent attached to both a hydrophilic and a hydrophobic region of the hydrogel. Amphiphilic hydrogel particles which are functionalised with an antimicrobial agent have a greater surface area and it is contended that they are more effective e.g., antibacterial agents, as they can expose a bacterial film to a greater portion of antimicrobial agent than a flat solid hydrogel.

The particles of amphiphilic hydrogel may be dispersed in a solution such as an aqueous solution. For example, the particles of amphiphilic hydrogel may be dispersed in a saline solution. As can be seen in Fig. 5 the performance of the antimicrobial hydrogel has been to still have an antimicrobial effect after 10 weeks in PBS. The particles of amphiphilic hydrogel may be dispersed in a biocompatible buffer, that is, a buffer which is non-toxic to cells. As opposed to many hydrogels, due to the amphiphilicity of the hydrogel the hydrogel particles may be dispersed in a non-aqueous solution, such as a non-polar solvent such as essential oil-based systems and in combination with alcohol.

The particles in the dispersion are swollen, that is, they have absorbed solution. However, as opposed to other hydrogel dispersions they are truly a suspension of discrete hydrogel particles in a solution, and not simply a hydrogel. A hydrogel may sometimes be described itself as a dispersion or suspension itself, as some hydrogels comprise discrete particles which swell and therein form the hydrogel. In this case the composition comprises a plurality of crosslinked hydrogel particles separate from each other, and separate from the continuous medium, e.g., aqueous solution.

The solution, and the particles dispersed in a solution are sprayable. A spray device may comprise a spraying mechanism, such as a manual spray pump, and a solution comprising a plurality of amphiphilic hydrogel particles as described above, wherein an antimicrobial agent, such as an antimicrobial peptide is covalently attached to the hydrophilic and/or the hydrophobic regions of the particles.

A spray device is shown schematically in Fig. 9. The device comprises a solution 100, for example an aqueous solution, such as a saline solution. The device further comprises a membrane 200 separating the solution from a plurality of antimicrobial hydrogel particles 300. Prior to a first use, an agitation process 500, such as shaking the device causes the membrane 200 to disintegrate and for the hydrogel particles to spread in the solution 100 forming a suspension of hydrogel particles 300. Such a device has enhanced storage stability as the hydrogel particles 300 are not stored in suspension, but maintained separately until the device is ready for use.

Spraying the amphiphilic hydrogel has advantages over placing a solid piece of hydrogel on a wound. The coverage is improved as the sprayed particles can cover an irregular surface better than a solid material. Furthermore, the spray can be used to rapidly cover a wound region without being held in place by additional bandages etc. The spray may therefore be ideal for use as an acute wound treatment.

The sprayable composition of antimicrobial amphiphilic hydrogel particles is furthermore an ideal coating medium for surgical or medical device, such as a stent, a catheter, a skin graft, a contact lens, personal hygiene articles, nappies, a wound dressing, an ostomy dressing, ostomy baseplate, incision film, surgical drape, a patch, a bandage, a band-aid, a plaster, an adhesive, an adhesive tape, an adhesive plaster, a sticking-plaster, and a court-plaster, or any combination thereof. A manufacturing or treatment process for any of the above listed devices may comprise a step of spraying the antimicrobial amphiphilic hydrogel particles on to the surface of the device and may include post treatments such as drying or scrubbing or wiping. The surface sprayed with the particles may be a surface which is intended to contact the human body, or bodily fluids.

Surprisingly, the present inventors have found that the antimicrobial amphiphilic hydrogel may be used for inducing and/or enhancing haemostasis of a wound. The antimicrobial hydrogel may therefore be used for increasing and/or accelerating blood coagulation and/or blood clot formation in a wound. As described in the experimental section and shown in Fig. 10 the antimicrobial hydrogel formed clearly visible clots in comparison to control samples. The acceleration of haemostasis may be in mammals, for example, haemostasis of human blood as shown in Experiment 1 may be accelerated.

As described in the background section, an antimicrobial amphiphilic hydrogel is known from WO 2019/074422 A1. However, that document is silent with regards to additional uses of the amphiphilic hydrogel beyond antimicrobial treatment of wounds. There is no suggestion that the antimicrobial hydrogel may be used to accelerate the process of haemostasis. Furthermore, not all wounds bleed such that haemostasis occurs. Wounds such as burns, and pressure sores do not bleed but are susceptible to bacterial and other microbial infections. This distinction does not appear to be considered in the referenced document as it does not relate to bleeding wounds. Therefore, the known therapeutic application from WO 2019/074422 A1, that is infection prevention or treatment, is not only different to enhancing or accelerating haemostasis, but is related to a general population of patients with wounds, rather than the specific target population of wounds which bleed.

The antimicrobial amphiphilic hydrogel may be especially useful for inducing or accelerating haemostasis in patients with a coagulopathy, i.e., a bleeding disorder in which the ability of the blood to coagulate is impaired.

As is shown in Fig. 7 the haemostatic action of the antimicrobial hydrogel is indeed a biochemical interaction with the body. The hydrogel alone did not cause a significant nor substantial reduction in the count of platelets in the measured sample.

The antimicrobial amphiphilic hydrogel may be used for simultaneously inducing haemostasis and inhibiting the growth of bacteria at and/or in a wound. The combination of the experimentally confirmed characteristics of serum stability and enhanced haemostatic effect make the antimicrobial amphiphilic hydrogel an ideal wound dressing for use for inducing haemostasis in a wound.

A method for treating a bleeding wound may comprise: placing an antimicrobial amphiphilic hydrogel according to the present description on a bleeding wound; thereby forming a blood clot at the interface between the antimicrobial amphiphilic hydrogel and blood; and, thereby substantially limiting, or hindering the growth of bacteria at the wound.

The sprayable dispersion of antimicrobial amphiphilic hydrogel particles may be ideally used for combined antimicrobial and haemostatic treatment. That is, the sprayable dispersion is ideal for use as a combined antimicrobial and haemostatic wound-care composition. As the hydrogel particles have improved surface coverage of irregular shapes, which is common for a wound, the hydrogel particles, are expected to have improved antimicrobial and haemostatic effects compared to a substantially flat sheet of amphiphilic hydrogel. Furthermore, the hydrogel is non-biodegradable and antimicrobial agents may maintain their effect for several days as shown in Fig. 6. This is especially relevant in a device for use in haemostasis of a bleeding wound, where the device will inherently be subject to a greater amount of contact with bodily fluids than in treatment of a non-bleeding wound.

The results provided herein suggest that a proline arginine-rich end leucine-rich repeat protein, PRELP, derived antimicrobial peptide, is surprisingly useful in haemostasis of a wound. For example, as shown in the results section, RRP9W4, has been shown to have a haemostatic effect. The PRELP derived antimicrobial peptide may be applied to a substrate.

Although, the present invention has been described above with reference to specific embodiments, it is not intended to be limited to the specific form set forth herein. Rather, the invention is limited only by the accompanying claims.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc. do not preclude a plurality. Reference signs in the claims are provided merely as a clarifying example and shall not be construed as limiting the scope of the claims in any way.

### Experimental section

The following examples are mere examples and should by no means be interpreted to limit the scope of the invention. Rather, the invention is limited only by the accompanying claims. Example 1 and 3 show effects of the composition but are not in the form of suspension of particles and thus not according to the invention as claimed.

### Experiment 1

### Manufacturing of amphiphilic hydrogels

Cross-linked amphiphilic hydrogels were prepared according to the method in Experiment 1 in WO 2019/074422 A1 for Pluronic^{®} F127. The reaction scheme is shown in Fig. 1. Briefly, a mixture of Pluronic F-127 (30 wt.%) and water (70 wt.%) was made to form a micellar cubic liquid crystalline phase. Irgacure 2959 was added to the mixture of Pluronic F-127 (2 wt.%) as a photoinitiator. Mixing was performed in 20ml glass vials manually using a spatula until a thick and homogenous gel formed. The gels were spread onto glass slides and kept in a sealed container overnight to set into correlating phase. The gels were then UV polymerized (90 W, *λ*= 252nm) for 10 min to form a flexible polymeric hydrogel with a thickness of 4-5 mm. The gels were cut into desired shapes and washed in mili-Q water for 48h to remove the unwanted by-products and get into their fully swollen shape before further analysis and AMP attachment.

Pluronic F127 (EO₁₀₀PO₇₀EO₁₀₀) was chemically functionalized with polymerizable diacrylate head groups as shown in WO 2019/074422 A1 Experiment 1 and the modified polymer was used for manufacturing of crosslinked F127 hydrogels for AMP modification.

### AMP immobilisation on amphiphilic hydrogels

AMPs were covalently immobilised to the hydrogels according to the submersion method described in Experiment 1 in WO 2019/074422 A1. A control amphiphilic hydrogel was prepared without any AMPs. The reaction scheme is shown in Fig. 2. Briefly, a solution of antimicrobial peptide (AMP) RRPRPRPRPWWWW-NH2 (RRP9W4N, Red Glead Discovery AB, Lund, Sweden) was prepared in sterilized water to a concentration of 200 µM. For covalent attachment of AMP to the hydrogels, prior to AMP modification, the clean hydrogels were submerged into a solution of 1-ethyl-3-(3-dimethylaminopropyl) carbodiimide hydrochloride (EDC) and N-Hydroxysuccinimide (NHS) mixed in MES buffer (pH 6) at a final concentration of 2 mg/ml and were allowed to react for 30 min on slow shake at room temperature. Hydrogels were then washed 3 times in PBS (pH 7.4) and suspended in 1ml of 200 µM AMP solution in sterilized water for 2 h in RT. The surfaces were washed 3 times with sterilized water to remove unreacted peptides and used for all the tests performed in this work.

### Blood coagulation test

Blood collecting Eppendorf tubes, pipette tips and loop tubing to draw the blood, were heparinized to avoid unwanted blood activation. Heparinization was done according to Corline method (Corline Biomedical AB, Uppsala, Sweden) which is done by a layer-by-layer assembly method with alternating incubation with a polymeric amine and a heparin conjugate to obtain a double-coated heparin layer. Fresh blood from 2 healthy volunteers was collected in heparinized tubes containing 1 IU/ml of Heparin solution ( Leo Pharma A/S, Ballerup, Denmark). The blood was used fresh after sampling. 1 ml of blood was collected in an Eppendorf tube with 4 mM EDTA to be used as a reference point (named initial).

Samples were conditioned by adding 1 ml of PBS and were shaken at 600 rpm for 30 min prior to the experiment. The hydrogels (control and AMP modified) were placed in Eppendorf test tubes. 100 µL of PBS were added in order to soak the samples. And then 1 ml of fresh blood added to each tube. The tubes were then rotated vertically for 60min in 37°C incubator. As blank controls, 1ml of blood was added to an Eppendorf tube without any hydrogels and treated with the same conditions. After the experiments the blood was carefully collected from the tubes and mixed with EDTA giving a final concentration of 4nM. The number of platelets was determined using a Sysmex XP-300 haematology analyser (Kobe Japan) directly after the experiment. Samples were run in duplicate with blood from each donor.

### Results and discussion

One of the most notable features of a proper wound management, is to control the bleeding. Blood coagulation involves formation of a fibrin clot in the presence of the wound which can trap the platelets and release inflammatory chemokines. That stage will create lateral responses to activate inflammatory responses and subsequent wound healing. Therefore we investigated how the presence of AMPs in the hydrogels can affect the blood coagulation. We performed a whole blood test using fresh blood from 2 different donors and quantified the number of platelets before and after 1h exposure to blood. A very visible clot formation was observed onto AMP-hydrogel compared to the ones without AMP. As expected from the observations, the results from platelets counting (shown in Fig. 7) showed significantly lower number of platelets in the presence of AMPs. The surprising results suggest that using AMP-hydrogel as a wound patch can accelerate blood coagulation and clot formation on a bleeding wound. That can be considered as a desired additional property introduced by AMP-hydrogels apart from the superior antibacterial effect.

The blood clotting effect is especially surprising as RRP9W4N is a proline arginine-rich end leucine-rich repeat protein, PRELP, derived antimicrobial peptide, and similar in many respects to LL-37, whereas LL-37 has been shown not to affect plasmatic coagulation (Harm, S et al., Blood Compatibility - An Important but Often Forgotten Aspect of the Characterization of Antimicrobial Peptides for Clinical Application, Int. J. Mol. Sci. 2019, 20, 5426; doi: 10.3390/ijms20215426).

### Experiment 2

### Sprayable dispersion of amphiphilic hydrogel

A sprayable formulation of granulated hydrogel was prepared according to the following procedure.

DA-F127 was synthesised as per Experiment 1 in WO 2019/074422 A1 for Pluronic^{®} F127, the DA-F127 was mixed with water with the composition 30 wt% Pluronic^{®} and 70 wt% water. After mixing, the initiator Irgacure^{®} 2959 (1-[4-(2-Hydroxyethoxy)-phenyl]-2-hydroxy-2-methyl-1-propane-1-one) was added to the formed gel with an amount corresponding to 0,5 wt% of the Pluronic^{®} in the gel. The gel was placed in a fridge for at least two days. Following cold storage, the gel was cross-linked at 365 nm for 6 minutes. The now cross-linked hydrogels were washed in water for at least two days.

After washing, the hydrogels were grinded to a rough paste with pestle and mortar. The paste was then put in water and an Ultra-Turrax^{®} disperser was used to get a finer particle size and distribution. The obtained solution was then stable for experiments to be performed at convenience.

A known weight of the particles (in swollen form) was obtained by suction filtration and then by putting the granules in a 15 ml falcon tube. This was normally around 2 grams. In order to activate the particles, 10 ml of newly prepared EDC/NHS (2mg/ml) in MES buffer was added to the falcon tube. The tube was sonicated for a few minutes and then put on a shaking plate for 30 minutes. The solution was then suction filtered and washed with water to separate the particles and to wash away the excess EDC/NHS. The granules were then weighed to record any loss and then 10 ml of 400 µM AMP (solved in PBS) was added to the granules. This was also sonicated quickly and then put on a shaking plate for about 2 hours.

The solution was once again suction filtered and washed with 30 ml of water, but this time the washing solution was recovered in order to measure the amount of peptide still left after activation. The granules are now activated and can be weighed and put in a solution to get a known concentration for further experiments.

### Antibacterial effect against S. aureus on agar plates

200 mg of each of the control granules (no activation) and the AMP-activated granules were put in separate Eppendorf tubes (4 each per round) and 180 µl of PBS solution was added to each tube. The tubes were then centrifuged shortly to get all the particles into the solution which was followed by sonication in water bath to redistribute the particles.

Agar plates were streaked with a culture of *S. aureus* at a concentration of 10⁸ CFU (colony forming units)/ml and left to dry for some minutes. The solutions of the Eppendorf tubes were then placed on top of the agar plates with one control and one AMP-activated version on each agar plate. The solution was gently spread on top of the agar plates to get a relatively uniform spread and thickness of the solutions each time. The agar plates were left open to air dry for some minutes until no liquid was observed around the granules so that the solution stayed in place on top of the agar after inversion. The agar plates were then incubated upside down overnight (~15 hours).

The following day, CFU experiments were done by taking a biopsy punch in the centre of each 'spread' which was put in Eppendorf tubes containing 1 ml of PBS. The tubes were vortexed and shaken for at least 10 minutes. A serial dilution of steps of 10 was then performed and 10 µl drops of suitable dilutions were put on agar plates and incubated for around 15 hours (until colonies were easy to count).

### Results and discussion

Fig. 8 shows an example of how the plates looked after the incubation overnight. The difference in colour/texture is most likely due to much less bacteria present in/beneath the AMP-activated granules. The colour difference visible in the pre-conversion archive. The black and white drawings as published show the different texture. It's also easy to see that bacteria have grown on the spots were coverage was bad for the AMP-activated particles, that is, where the sprayable particles were not spread on the plate.

**Table 1: CFU/ml count from the agar studies using S. aureus. n=12**

| Type | CFU/ml | AMP-activated efficiency |
|---|---|---|
| Control | 2.51 ± 1.03 x10⁸ | 99.9993% less bacteria on AMP-activated compared to the control |
| AMP-activated | 1.67 ± 2.42 x10⁴ | |

The results from the CFU is presented in Table 1. Where the CFU corresponds to the number of bacteria in the 1 ml of PBS that the biopsies were washed in. The results clearly show a significant reduction of about 99.993 % when the AMP-activated granules are applied compared to the control ones.

UV-vis measurements of the AMP residues after washing the granules showed that 1.7 grams of the particles took up about 5.7 mg of peptides. This compares to 0.07 mg of peptides for 0.07 g of solid sheet formed amphiphilic hydrogel. The particles took up about 3.35 times as much peptide compared to the solid hydrogel in non-particle/granular form.

To show the proof of concept, particles that were solved in PBS were placed in a simple mechanical spray bottle and sprayed on to a surface. The particles were sprayable and covered a surface area of approximately 25 cm² rapidly and relatively uniformly. Thickness uniformity was inspected visually.

### Experiment 3

### Blood coagulation on hydrogel samples with different peptides

Hydrogels were functionalized with 4 different types of antimicrobial peptides (AMPs). The AMPs used were PGLa, LL-37, Temporin B and RRP9W4. As control hydrogels without any attached AMPs were used.

Fresh citrated Horse blood was purchased from Håtunalab AB, Sweden and used to show how if presence of AMPs can induce clotting. In an Eppendorf tube, 1 ml of blood with 0.1 ml of 250 Mm calcium chloride (to reverse the citrate anticoagulant) was added and mixed.

Samples were placed in 24 well plate and conditioned by adding 1 ml PBS to the hydrogels. After 15 minutes the samples were transferred to the Eppendorf tubes containing the blood. The tubes were then placed on shaker and incubated for 60 min at 37 °C.

As blank controls, 1 ml of blood was added to an Eppendorf tube without any hydrogels and treated with the same conditions. After the experiments, the blood was carefully collected from the tubes and the hydrogels were taken out of the tube carefully using a tweezer (at this point some coagulation was observed to the side of the test tube walls), the hydrogels were washed three times with flushing PBS to remove the unattached clots and other blood cells before imaging. Samples were run in duplicate.

### Results and discussion

This test was performed to investigate that how the presence of AMPs on the hydrogels affect blood coagulation. Here, whole horse blood test was performed to see if any observable clot can form onto hydrogels with different peptides covalently attached to it. A large visible clot was observed on hydrogels with RRP9W4, whereas no clot formation was observed on hydrogels with the other AMPs (PGLa, LL-37 and Temporin B) and the control hydrogels without any AMPs.

In this test only the clots that was firmly attached to the hydrogels surfaces was considered as clots induced by hydrogel-blood contact. Other clots formed during this test was mainly attached to the test tube walls or detached easily from the surface, see the table below, where a * indicates formation of a clot fixed to the hydrogel.

| **AMP-Hydrogels** | **Observations** |
|---|---|
| RRP9W4* | Big clot with firm attachment onto the surface |
| | Did not detach by PBS flushing and not mechanically removable by tweezer. |
| PGLa | No visible clot was observed. |
| LL-37 | No visible clot was observed. |
| Temporin B | Small clots loosely attached and washed away by PBS flushing. |
| Control- no AMP | No visible clot was observed. |

Due to the simple and qualitative nature of experiment 3, the results above are not considered conclusive. However, as PGLa, LL-37 and Temporin B, all of which are similar to RRP9W4 in terms of linear structure, net charge, and hydrophobicity, did not form a strong clot on the surface of the hydrogel, this supports the surprising nature of the result of RRP9W4. In particular, it was noted that Temporin B and RRP9W4 have the same length (13 amino acids) and a similar hydrophobicity (4.22 kcal/mol and 9.15 kcal/mol respectively) yet showed different clotting results on the hydrogel. Furthermore, the net charge on RRP9W4 and LL-37 (+6 and +6 respectively) are similar yet, in accordance with previous results LL-37 showed no clotting effect whilst RRP9W4 continued to show the surprising clotting effect.

## Claims

1. An antimicrobial hydrogel composition comprising:
- a first cross-linkable amphiphilic component, the first amphiphilic component, in its chemically cross-linked state, being a lyotropic liquid crystal and having an ordered nanostructure of hydrophobic and hydrophilic domains,
- an antimicrobial agent covalently attached to the hydrophilic and/or hydrophobic domains,
wherein the cross-linkable amphiphilic component is a cross-linked lyotropic liquid crystal, and wherein the composition is a suspension of cross-linked hydrogel particles in a solution.

2. The antimicrobial hydrogel composition according to claim 1, wherein the antimicrobial agent is a substantially amphiphilic antimicrobial agent covalently immobilized on the hydrophilic domains and, optionally, hydrophobic domains of the first amphiphilic component.

3. The antimicrobial hydrogel composition according to claim 2, wherein the antimicrobial agent is an antimicrobial peptide.

4. The antimicrobial hydrogel composition according to claim 3, wherein the antimicrobial peptide is proline arginine-rich end leucine-rich repeat protein, PRELP, derived antimicrobial peptide, having a length of less than 37 amino acids and a stretch of at least one, such as at least three, hydrophobic amino acids forming a hydrophobic region for interaction with the hydrophobic regions of the hydrogel.

5. The antimicrobial hydrogel composition according to claims 5 or 6, wherein the antimicrobial peptide is an antimicrobial peptide comprising less than 20 amino acids, and comprising a sequence having at least 90%, such as 95%, identity to the amino acid sequence RRPRPRPRP.

6. The antimicrobial hydrogel composition according to any of claims 5 to 7, wherein the antimicrobial peptide is RRP9W4N.

7. A method for producing an antimicrobial amphiphilic hydrogel suspension, comprising:
- providing a first cross-linkable amphiphilic component;
- forming a lyotropic liquid crystal, LLC, amphiphilic hydrogel from the cross-linkable amphiphilic component;
- cross-linking the LLC amphiphilic hydrogel to form a chemically cross-linked solid LLC hydrogel;
- processing the chemically cross-linked solid LLC hydrogel to form hydrogel particles;
- attaching, covalently at the hydrophilic and/or hydrophobic regions of the amphiphilic hydrogel particles, an antimicrobial agent to the hydrogel particles; and,
- providing the cross-linked antimicrobial particles in a continuous medium.

8. The method according to claim 7, wherein the processing to form hydrogel particles comprises grinding the solid LLC hydrogel to form the hydrogel particles.

9. The method according to claim 7 or 8, wherein the antimicrobial agent is an antimicrobial peptide.

10. The method according to claim 9, wherein the antimicrobial peptide is a proline arginine-rich end leucine-rich repeat protein, PRELP, derived antimicrobial peptide, having a length of less than 37 amino acids and a stretch of at least one, such as least three, hydrophobic amino acids forming a hydrophobic region for interaction with the hydrophobic regions of the hydrogel.

11. The method according to claim 9 or 10, wherein the antimicrobial peptide is an antimicrobial peptide comprising less than 20 amino acids, and comprises a sequence having at least 90%, such as 95%, identity to the amino acid sequence RRPRPRPRP.

12. The method according to any of claims 9 to 11, wherein the antimicrobial peptide is RRP9W4N.

13. A device comprising at least one surface for contact with a human body, and/or bodily fluids, wherein at least a portion of the surface comprises a coating of antimicrobial amphiphilic hydrogel composition according to any of claims 1 to 6.

14. An antimicrobial hydrogel composition obtainable via the method according to any of claims 7 to 12.

## Patentansprüche

1. Antimikrobielle Hydrogelzusammensetzung, umfassend:
- eine erste vernetzbare amphiphile Komponente, wobei die erste amphiphile Komponente in ihrem chemisch vernetzten Zustand ein lyotroper Flüssigkristall ist und eine geordnete Nanostruktur aus hydrophoben und hydrophilen Domänen aufweist,
- ein kovalent an die hydrophilen und/oder hydrophoben Domänen gebundenes antimikrobielles Mittel,
wobei die vernetzbare amphiphile Komponente ein vernetzter lyotroper Flüssigkristall ist, und wobei die Zusammensetzung eine Suspension von vernetzten Hydrogelpartikeln in einer Lösung ist.

2. Antimikrobielle Hydrogelzusammensetzung nach Anspruch 1, wobei das antimikrobielle Mittel ein im Wesentlichen amphiphiles antimikrobielles Mittel ist, das kovalent an den hydrophilen Domänen und gegebenenfalls hydrophoben Domänen der ersten amphiphilen Komponente immobilisiert ist.

3. Antimikrobielle Hydrogelzusammensetzung nach Anspruch 2, wobei das antimikrobielle Mittel ein antimikrobielles Peptid ist.

4. Antimikrobielle Hydrogeldispersion nach Anspruch 3, wobei das antimikrobielle Peptid ein von prolin-arginin-reichem End-Leucin-reichem Repeat-Protein, PRELP, abgeleitetes antimikrobielles Peptid ist, das eine Länge von weniger als 37 Aminosäuren und einen Abschnitt von mindestens einer, wie beispielsweise mindestens drei, hydrophoben Aminosäuren aufweist, der eine hydrophobe Region zur Wechselwirkung mit den hydrophoben Regionen des Hydrogels bildet.

5. Antimikrobielle Hydrogeldispersion nach Anspruch 5 oder 6, wobei das antimikrobielle Peptid ein antimikrobielles Peptid ist, das weniger als 20 Aminosäuren umfasst und eine Sequenz umfasst, die zu mindestens 90 %, wie beispielsweise 95 %, Identität mit der Aminosäuresequenz RRPRPRPRP aufweist.

6. Antimikrobielle Hydrogeldispersion nach einem der Ansprüche 5 bis 7, wobei das antimikrobielle Peptid RRP9W4N ist.

7. Verfahren zur Herstellung einer antimikrobiellen amphiphilen Hydrogelsuspension, umfassend:
- Bereitstellen einer ersten vernetzbaren amphiphilen Komponente;
- Bilden eines lyotropen flüssigkristallinen, LLC-, amphiphilen Hydrogels aus der vernetzbaren amphiphilen Komponente;
- Vernetzen des amphiphilen LLC-Hydrogels zur Bildung eines chemisch vernetzten festen LLC-Hydrogels;
- Verarbeiten des chemisch vernetzten festen LLC-Hydrogels zur Bildung von Hydrogelpartikeln;
- Binden, kovalent an die hydrophilen und/oder hydrophoben Regionen der amphiphilen Hydrogelpartikel, eines antimikrobiellen Mittels an die Hydrogelpartikel; und
- Bereitstellen der vernetzten antimikrobiellen Partikel in einem kontinuierlichen Medium.

8. Verfahren nach Anspruch 7, wobei die Verarbeitung zur Bildung von Hydrogelpartikeln das Mahlen des festen LLC-Hydrogels umfasst, um die Hydrogelpartikel zu bilden.

9. Verfahren nach Anspruch 7 oder 8, wobei das antimikrobielle Mittel ein antimikrobielles Peptid ist.

10. Verfahren nach Anspruch 9, wobei das antimikrobielle Peptid ein von einem prolin- und argininreichen End-Leucin-reichen Repeat-Protein, PRELP, abgeleitetes antimikrobielles Peptid ist, das eine Länge von weniger als 37 Aminosäuren und einen Abschnitt von mindestens einer, wie mindestens drei, hydrophoben Aminosäuren aufweist, der eine hydrophobe Region zur Wechselwirkung mit den hydrophoben Regionen des Hydrogels bildet.

11. Verfahren nach Anspruch 9 oder 10, wobei das antimikrobielle Peptid ein antimikrobielles Peptid ist, das weniger als 20 Aminosäuren umfasst, und eine Sequenz umfasst, die zu mindestens 90 %, wie beispielsweise 95 %, Identität mit der Aminosäuresequenz RRPRPRPRP aufweist.

12. Verfahren nach einem der Ansprüche 9 bis 11, wobei das antimikrobielle Peptid RRP9W4N ist.

13. Vorrichtung, umfassend mindestens eine Oberfläche für den Kontakt mit einem menschlichen Körper und/oder Körperflüssigkeiten, wobei mindestens ein Teil der Oberfläche eine Beschichtung aus einer antimikrobiellen amphiphilen Hydrogelzusammensetzung nach einem der Ansprüche 1 bis 6 umfasst.

14. Antimikrobielle Hydrogelzusammensetzung, erhältlich durch das Verfahren nach einem der Ansprüche 7 bis 12.

## Revendications

1. Composition d'hydrogel antimicrobien comprenant :
- un premier composant amphiphile réticulable, le premier composant amphiphile, dans son état chimiquement réticulé, étant un cristal liquide lyotrope et ayant une nanostructure ordonnée de domaines hydrophobes et hydrophiles,
- un agent antimicrobien lié de manière covalente aux domaines hydrophiles et/ou hydrophobes,
dans laquelle le composant amphiphile réticulable est un cristal liquide lyotrope réticulé, et dans laquelle la composition est une suspension de particules d'hydrogel réticulé dans une solution.

2. Composition d'hydrogel antimicrobien selon la revendication 1, dans laquelle l'agent antimicrobien est un agent antimicrobien sensiblement amphiphile immobilisé de manière covalente sur les domaines hydrophiles et, éventuellement, les domaines hydrophobes du premier composant amphiphile.

3. Composition d'hydrogel antimicrobien selon la revendication 2, dans laquelle l'agent antimicrobien est un peptide antimicrobien.

4. Dispersion d'hydrogel antimicrobien selon la revendication 3, dans laquelle le peptide antimicrobien est un peptide antimicrobien dérivé de la protéine à répétition riche en leucine et à extrémité riche en proline/arginine, PRELP, ayant une longueur inférieure à 37 acides aminés et un segment d'au moins un, par exemple d'au moins trois, acides aminés hydrophobes formant une région hydrophobe pour l'interaction avec les régions hydrophobes de l'hydrogel.

5. Dispersion d'hydrogel antimicrobien selon la revendication 5 ou 6, dans laquelle le peptide antimicrobien est un peptide antimicrobien comprenant moins de 20 acides aminés, et comprenant une séquence ayant au moins 90 %, par exemple 95 %, d'identité avec la séquence d'acides aminés RRPRPRPRP.

6. Dispersion d'hydrogel antimicrobien selon l'une quelconque des revendications 5 à 7, dans laquelle le peptide antimicrobien est RRP9W4N.

7. Procédé pour la production d'une suspension d'hydrogel amphiphile antimicrobien, comprenant :
- la fourniture d'un premier composant amphiphile réticulable ;
- la formation d'un hydrogel amphiphile à cristal liquide lyotrope, LLC, à partir du composant amphiphile réticulable ;
- la réticulation de l'hydrogel amphiphile à LLC pour former un hydrogel à LLC solide réticulé chimiquement ;
- le traitement de l'hydrogel à LLC solide réticulé chimiquement pour former des particules d'hydrogel ;
- la liaison, de manière covalente au niveau des régions hydrophiles et/ou hydrophobes des particules d'hydrogel amphiphile, d'un agent antimicrobien aux particules d'hydrogel ; et,
- la fourniture des particules antimicrobiennes réticulées dans un milieu continu.

8. Procédé selon la revendication 7, dans lequel le traitement pour former des particules d'hydrogel comprend le broyage de l'hydrogel à LLC solide pour former les particules d'hydrogel.

9. Procédé selon la revendication 7 ou 8, dans lequel l'agent antimicrobien est un peptide antimicrobien.

10. Procédé selon la revendication 9, dans lequel le peptide antimicrobien est un peptide antimicrobien dérivé de la protéine à répétition riche en leucine et à extrémité riche en proline/arginine, PRELP, ayant une longueur inférieure à 37 acides aminés et un segment d'au moins un, par exemple d'au moins trois, acides aminés hydrophobes formant une région hydrophobe pour l'interaction avec les régions hydrophobes de l'hydrogel.

11. Procédé selon la revendication 9 ou 10, dans lequel le peptide antimicrobien est un peptide antimicrobien comprenant moins de 20 acides aminés, et comprend une séquence ayant au moins 90 %, par exemple 95 %, d'identité avec la séquence d'acides aminés RRPRPRPRP.

12. Procédé selon l'une quelconque des revendications 9 à 11, dans lequel le peptide antimicrobien est RRP9W4N.

13. Dispositif comprenant au moins une surface destinée à entrer en contact avec un corps humain, et/ou des fluides corporels, dans lequel au moins une partie de la surface comprend un revêtement d'une composition d'hydrogel amphiphile antimicrobien selon l'une quelconque des revendications 1 à 6.

14. Composition d'hydrogel antimicrobien pouvant être obtenue par l'intermédiaire du procédé selon l'une quelconque des revendications 7 à 12.
